# EUROPEAN PATENT SPECIFICATION

(11) **EP 4 062 961 B1**
(45) Date of publication and mention of the grant of the patent: **19.02.2025**
(21) Application number: 21904624.0
(22) Date of filing: 14.10.2021
(51) Int. Cl.: A61M 16/04

(54) **SPUTUM CONTAINER FOR TRACHEOSTOMY PATIENT**
SPUTUM-BEHÄLTER FÜR TRACHEOSTOMIEPATIENTEN
RÉCIPIENT D'EXPECTORATIONS POUR PATIENT SOUS TRACHÉOSTOMIE

(30) Priority: 23.12.2020 KR 20200182209; 28.05.2021 KR 20210069114; 10.09.2021 KR 20210120959
(43) Date of publication of application: 28.09.2022
(73) Proprietor: Oh, Hyuck Gun, Seoul 05720 (KR); Yoo, Tae Hyoung, Yongin-si, Gyeonggi-do 17033 (KR)
(72) Inventor: Oh, Hyuck Gun, Seoul 05720 (KR); Yoo, Tae Hyoung, Yongin-si, Gyeonggi-do 17033 (KR)
(74) Representative: BCKIP Part mbB
(86) International application number: PCT/KR2021/014199
(87) International publication number: WO 2022/139137

(56) References cited:
- CN-U- 202 777 129
- CN-U- 203 989 086
- JP-A- 2020 116 375
- KR-A- 20150 008 194
- KR-B1- 101 888 826
- KR-Y1- 200 151 570
- US-A1- 2006 207 602
- US-A1- 2014 102 441
- US-A1- 2018 078 723

## Description

### [Technical Field]

The present invention relates to a sputum container for a tracheostomy patient, which receives sputum discharged by a patient into whom a tracheostomy tube (intubation tube) is inserted, more specifically, to a sputum container for a tracheostomy patient, which allows a tracheostomy patient to breathe comfortably, prevents backflow from a sputum accommodation part, and is coupled to a tracheostomy tube.

### [Background Art]

A tracheostomy is a procedure which is performed when an upper part of a trachea, which is a path through which air flows in or out, is blocked, and respiratory failure is predicted, solves a problem of respiratory failure by opening a passage to a bronchus from the neck in order to secure an airway even when the upper trachea is blocked.

A tracheostoma is a portion which is formed by temporarily or permanently cutting a portion connecting the skin of the neck and an airway and inserting a tracheostomy tube thereinto to form a path in the human body for breathing, and a patient breathes and discharges secretions through the tracheostoma.

As described above, foreign substances, such as sputum or phlegm, in the human body are strongly unconsciously discharged to the outside of the human body from the lungs or airways of a tracheostomy patient through a tracheostomy tube inserted into the tracheostomy patient, but there are no tools or apparatuses to receive the foreign substances.

As a temporary measure for this situation in a conventional hospital, a paper cup is attached to the neck of a tracheostomy patient and receives discharged sputum, and the sputum discharged from the patient is splattered onto the face of a doctor or nurse, other patients in an intensive care unit, a curtain, a ceiling, and furniture.

Some cases, when a large amount of sputum is discharged, the sputum causes a situation in which it is difficult for a patient to breathe, and the sputum, which is strongly ejected in all directions, can cause secondary infections in medical staff (doctors, nurses, and the like) of an intensive care unit, and thus the medical staff of the intensive care unit complain about difficulties in a treatment environment due to tracheostomy patients.

Meanwhile, as related art, there is Korean Patent No. 10-2215855 (Registered on February 8, 2021, Title of invention: INTUBATION TUBE). This patent document discloses an intubation apparatus including an adaptor connected to an external oxygen supply apparatus, an oxygen supply tube having one end portion coupled to the adaptor and the other end portion in which an oxygen discharge port is formed, balloons including a first balloon and a second balloon which are coupled to an outer surface adjacent to the other end portion of the oxygen supply tube and operate independently of each other, a first balloon air supply tube though which air is supplied to the first balloon, and a second balloon air supply tube through which air is supplied to the second balloon, and each of the first balloon and the second balloon includes an oblique extension part surrounding the oxygen supply tube in an oblique direction with respect to an extension direction of the oxygen supply tube.

In addition, Korean Patent No. 10-2215835 (Registered on February 8, 2021, Title of invention: INTUBATION TUBE HAVING SELF CLEANING PROPERTY) discloses an intubation tube which has a self-cleaning property and includes a tube body longitudinally extending and including a breathing path and a suction path with a boundary wall interposed therebetween and in which an air flow of the inhalation or exhalation of a patient occurs through the breathing path, and the suction path is connected to an external suction apparatus to discharge phlegm of the patient to the outside of the tube body, and a through hole allowing the breathing path and the suction path to communicate with each other is formed in the boundary wall.

However, it seems that even the related art documents have no solutions for the above-described problems mentioned by the present applicant. That is, the related art does not disclose (i) a sputum container for a tracheostomy patient which receives sputum discharged by a patient into whom a tracheostomy tube (intubation tube) is inserted, (ii) a sputum container for a tracheostomy patient which allows a tracheostomy patient to breathe comfortably, prevents sputum from backflowing from an accommodation part, and is coupled to a tracheostomy tube, (iii) an apparatus capable of addressing secondary infections and treatment environment grievances of medical staff of an intensive care unit due to a situation in which it is difficult for a patient to breathe because a large amount of sputum is discharged from the tracheostomy patient and the sputum is strongly ejected out of the body, and iv) an apparatus capable of addressing a situation in which sputum from a tracheostomy patient is splattered onto a face of a doctor, nurse, or the like, other patients, and objects (curtains, ceilings, and furniture) because a paper cup is attached to a neck of the patient and receives the discharged sputum in the conventional hospital.

US 2014/102441 A1 discloses a closed automated suction device (CASD). The device is designed to overcome the need for manual suction and to facilitate strict asepsis in patients who are endotracheally intubated or tracheostomied. The device includes an outer tube with an inner tube concentrically nested within to define a cavity therebetween. The inner tube comprises a passage extended therethrough for providing ventilation, and the cavity between the inner and outer tubes is configured for communication with a vacuum source to provide aspiration.

US 2006/207602 A1 discloses an endotracheal tube assembly.

CN 203989086 U discloses a disposable sputum collector.

CN 202777129 U discloses a further sputum collecting container.

### [Disclosure of the Invention]

### [Technical Problem]

It is an object of the present invention to provide a sputum container for a tracheostomy patient, which receives sputum discharged from a patient into whom a tracheostomy tube (intubation tube) is inserted.

In addition, it is an object of the present invention to provide a sputum container for a tracheostomy patient, which allows a tracheostomy patient to breathe comfortably, prevents backflow from a sputum accommodation part, and is coupled to a tracheostomy tube.

In addition, it is an object of the present invention to provide a sputum container for a tracheostomy patient capable of addressing secondary infections and treatment environment grievances of medical staff of an intensive care unit due to a situation it is difficult for a patient to breathe because a large amount of sputum is discharged from the tracheostomy patient and the sputum is strongly ejected out of the body.

In addition, it is an object of the present invention to provide a sputum container for a tracheostomy patient capable of addressing a situation in which sputum from a tracheostomy patient is splattered onto a face of a doctor, nurse, or the like, other patients, and objects (curtains, ceilings, and furniture) because a paper cup is attached to a neck of the patient and receives the discharged sputum in the conventional hospital.

### [Technical Solution]

To solve the above problems, the present invention provides a sputum container for a tracheostomy patient in accordance with claim 1.

One aspect of the present invention provides a sputum container for a tracheostomy patient, which is applied to a tracheostomy patient, the sputum container including a sputum accommodation part which accommodates a patient's sputum and a tracheostomy tube coupling part which is provided at one upper side of the sputum accommodation part, is coupled to a tracheostomy tube which is a tracheostomy region of the patient and is disposed in a first direction, receives the sputum of the patient, and delivers the sputum to the sputum accommodation part.

The sputum container includes an oxygen supply and suction tube coupling part which is provided at one side of the tracheostomy tube coupling part in a second direction, which is a direction opposite to the first direction, and coupled to an oxygen supply tube or suction tube.

The tracheostomy tube coupling part may be formed to extend upward from the sputum accommodation part and have a shape bent in the first direction.

The sputum container may further include a sputum backflow prevention part which is formed to extend upward from the sputum accommodation part and includes one or more holes which are open in the first direction to prevent the sputum contained in the sputum accommodation part from flowing backward and allow air to flow into or out of the sputum container.

The sputum backflow prevention part may be provided as one or more sputum backflow prevention parts formed at at least any one side or each side of the tracheostomy tube coupling part on the sputum accommodation part.

The tracheostomy tube coupling part may be formed on an extension part formed to extend upward from the sputum accommodation part in the first direction, the oxygen supply and suction tube coupling part may be formed on the extension part formed to extend upward from the sputum accommodation part in the second direction, and the tracheostomy tube coupling part and the oxygen supply and suction tube coupling part may be integrally formed.

An insertion hole which is formed as an internal protruding type and into which an oxygen supply tube or suction tube is inserted may be formed to prevent the sputum from being discharged to the outside of the oxygen supply and suction tube coupling part.

The sputum container may further include an air entrance including a hole which opens in the second direction to smoothly supply air to the patient.

The air entrance may be formed as a grid structure to prevent, except air, foreign substances including insects, such as flies and mosquitos, and dust from being introduced into a bronchus.

The sputum accommodation part may have a cylindrical shape of which a cross-section has a circular shape.

The sputum container may include a communication part which is disposed between the sputum backflow prevention part and the tracheostomy tube coupling part and has a structure through which at least parts of the sputum backflow prevention part and the tracheostomy tube coupling part, which are positioned at levels higher than a level of a maximum sputum accommodating position in the sputum accommodation part, communicate with each other so that the air flowing into through the sputum backflow prevention part flows into an airway of the patient through the tracheostomy tube coupling part even when the sputum is maximally contained in the sputum accommodation part.

The maximum sputum accommodating position may be a position of a hole having a lowest height from the sputum accommodation part among the holes which are open in the sputum accommodation part in the first direction.

### [Advantageous Effects]

A sputum container for a tracheostomy patient according to the present invention has following effects.

First, a container which allows a tracheostomy patient to breathe comfortably and receives sputum discharged by the patient into whom a tracheostomy tube (intubation tube) is inserted at the same time is provided.

Second, sputum is prevented from pouring out of a container containing the sputum or backflowing no matter what position the tracheostomy patient wearing the container containing the sputum is in, that is, the tracheostomy patient is lying down, turning, sitting, or bowing his/her head.

When the sputum flows backward and returns to the trachea, respiratory difficulties, pneumonia, or the like can occur in a tracheostomy patient, and in some cases, serious problems leading to death can occur in critically ill patients with weak immunity. The sputum container for a tracheostomy patient according to the present invention can fundamentally prevent these problems.

Third, a temporary measure of attaching a paper cup to the neck of a tracheostomy patient can be addressed.

Fourth, a conventional situation in which sputum is unconsciously discharged (generally strongly expelled like a cough) from the lungs, trachea, or airways and splattered on faces of medical staff such as doctors or nurses can be prevented.

Fifth, a situation in which sputum is splattered on medical staff working in an intensive care unit in which a tracheostomy patient is hospitalized, other patients being treated in the same intensive care unit, and hospital room objects such as curtains, ceilings, and furniture can be prevented, and thus secondary infection or contamination problems in the hospital room and treatment environment grievances can be addressed.

### [Brief Description of the Drawings]

FIG. 1 is a side view of a sputum container (10) for a tracheostomy patient according to the present invention as seen in a direction d1.
FIG. 2 is a side view of the sputum container (10) for a tracheostomy patient according to the present invention as seen in a direction d2 which is a direction opposite to the direction d1 of FIG. 1.
FIG. 3 is a plan view of the sputum container (10) for a tracheostomy patient according to the present invention as seen from above.
FIG. 4 is a right side view of the sputum container (10) for a tracheostomy patient according to the present invention as seen from the right.
FIGS. 5 and 6 are plan views of a first portion (11) and a second portion (12) for manufacturing the sputum container (10) for a tracheostomy patient according to the present invention, wherein FIG. 5 is a plan view as seen in the direction d1, and FIG. 6 is a plan view as seen in the direction d2.
FIGS. 7 and 8 are perspective views of the first portion (11) and the second portion (12) for manufacturing the sputum container (10) for a tracheostomy patient according to the present invention, wherein FIG. 7 is a perspective view as seen in the direction d2, and FIG. 6 is a perspective view as seen in the direction d1.

### [Detailed Description of Exemplary Embodiments of the Invention]

Hereinafter, exemplary embodiments of the present invention will be described in more detail with reference to the accompanying drawings.

The embodiments described in this specification and configurations illustrated in the drawings are only exemplary embodiments.

This application claims domestic priority for two patent applications of the present applicant's Korean Patent Application No. 10-2020-0182209 (Application Date: December 23, 2020) and Korean Patent Application No. 10-2021-69114 (Application Date: May 26, 2021).

### (Sputum container for tracheostomy patient)

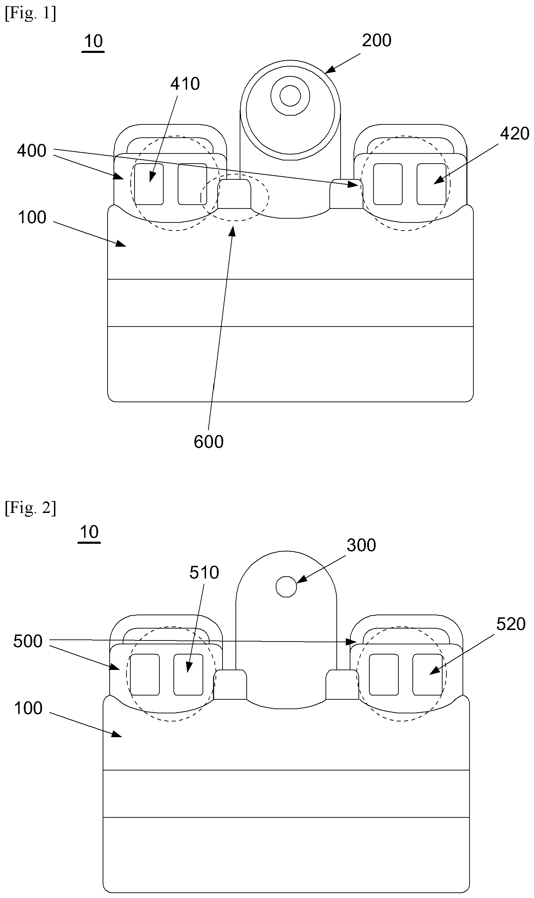

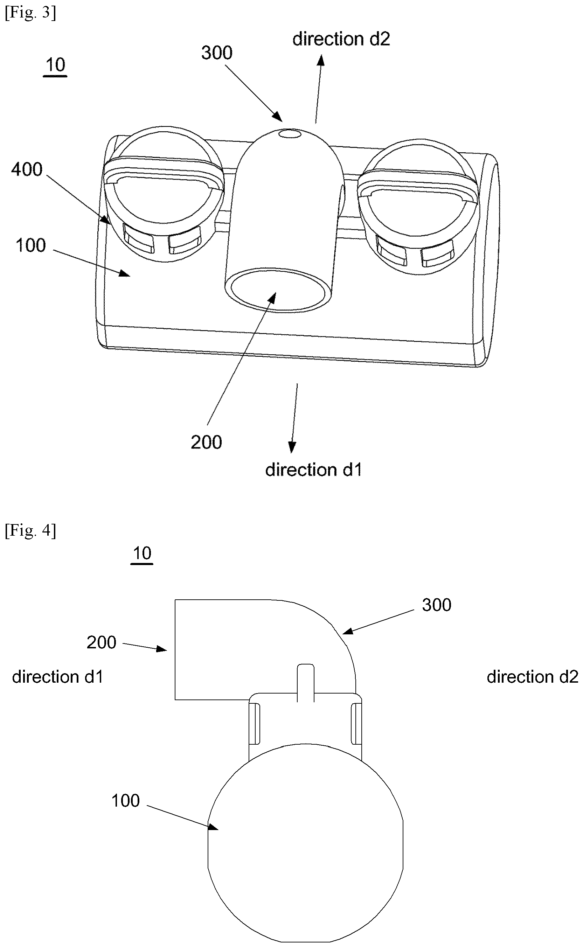

FIG. 1 is a side view of a sputum container 10 for a tracheostomy patient according to the present invention as seen in a direction d1, FIG. 2 is a side view of the sputum container 10 for a tracheostomy patient according to the present invention as seen in a direction d2 which is a direction opposite to the direction d1 of FIG. 1, FIG. 3 is a plan view of the sputum container 10 for a tracheostomy patient according to the present invention as seen from above, and FIG. 4 is a right side view of the sputum container 10 for a tracheostomy patient according to the present invention as seen from the right. Hereinafter, the present invention will be described with reference to the accompanying drawings.

As illustrated in FIGS. 1 to 4, the sputum container 10 for a tracheostomy patient according to one embodiment of the present invention applied to a tracheostomy patient includes a sputum accommodation part 100 and a tracheostomy tube coupling part 200.

The sputum accommodation part 100 is an accommodation part or container which accommodates sputum (phlegm) which is consciously or unconsciously discharged, spewed out, or flows from the lungs, airways, trachea, or the like, that is, from the body of a tracheostomy patient. A process of manufacturing the sputum accommodation part 100 having a cylindrical shape of which a cross section has a circular shape is simple, and thus manufacturing costs can be reduced.

In addition, the tracheostomy tube coupling part 200 is a part which is provided at one upper side of the sputum accommodation part 100, is coupled to a tracheostomy tube (not shown) which is a tracheostomy region of the patient in a first direction d1, receives sputum of the patient, delivers the sputum to the sputum accommodation part 100. As illustrated in the drawings (particularly, as illustrated in FIG. 3), the tracheostomy tube coupling part 200 may be formed in a shape extending upward from the sputum accommodation part 100 to be bent in the first direction d1. In this case, an uneven portion including protrusions and grooves may be provided on one portion, to which the tracheostomy tube is fitted, of an inner side or an outer side of an end of the tracheostomy tube coupling part 200, and thus fitting reliability can be improved.

Meanwhile, the present invention includes an oxygen supply and suction tube coupling part 300 which is provided at one side of the tracheostomy tube coupling part 200 in a second direction d2 opposite to the first direction d1 and coupled to an oxygen supply tube and/or a suction tube (not shown).

In addition, the present invention may further include a sputum backflow prevention part 400. The sputum backflow prevention part may be formed to extend upward from the sputum accommodation part and include one or more holes 410 and 420 which are open in the first direction d1 to prevent sputum contained in the sputum accommodation part 100 from flowing backward and allow air to flow into or out of the sputum container.

One or more sputum backflow prevention parts 400 may be formed on the sputum accommodation part 100 at both sides of the tracheostomy tube coupling part 200. Alternatively, one or more sputum backflow prevention parts 400 may be formed at either side of the tracheostomy tube coupling part 200. That is, there may be only the hole 410, there may be only the hole 420, or there may be the holes 410 and 420 at both sides.

In this case, the present invention may include a communication part 600 which is disposed between the sputum backflow prevention part 400 and the tracheostomy tube coupling part 200 and has a structure through which at least parts of the sputum backflow prevention part 400 and the tracheostomy tube coupling part 200, which are positioned at levels higher than a level of a maximum sputum accommodating position in the sputum accommodation part 100, communicate with each other so that air flowing into through the sputum backflow prevention part 400 flows into the airway of the patient through the tracheostomy tube coupling part even when the sputum is maximally accommodated in the sputum accommodation part 100. In this case, the maximum sputum accommodating position is a position of a hole having a lowest height from the sputum accommodation part among the holes 410 and 420 which are open in the first direction d1 in the sputum accommodation part 100.

In addition, the tracheostomy tube coupling part 200 and the oxygen supply and suction tube coupling part 300 may be integrally formed by forming the tracheostomy tube coupling part 200 in the first direction d1 of an extension part formed to extend upward from the sputum accommodation part 100 and forming the oxygen supply and suction tube coupling part 300 in the second direction d2 of the extension part formed to extend upward from the sputum accommodation part 100.

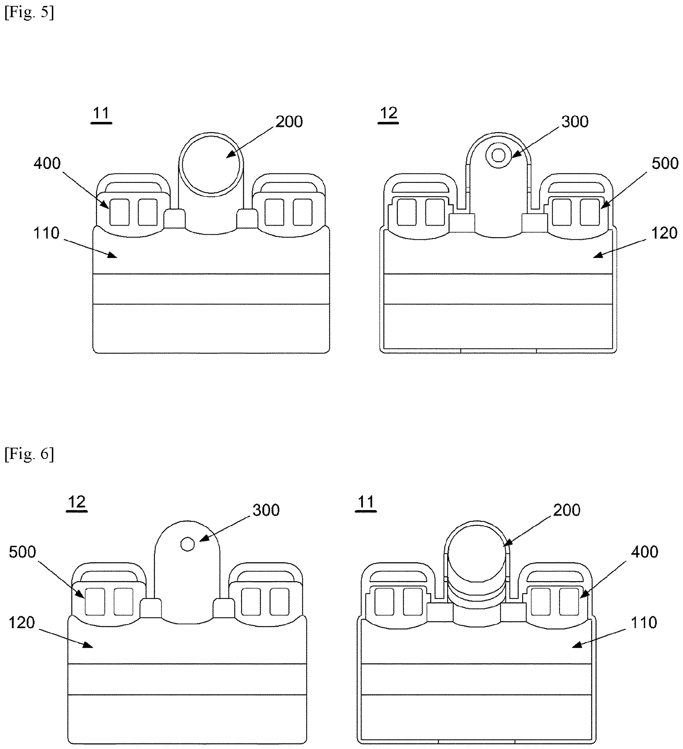

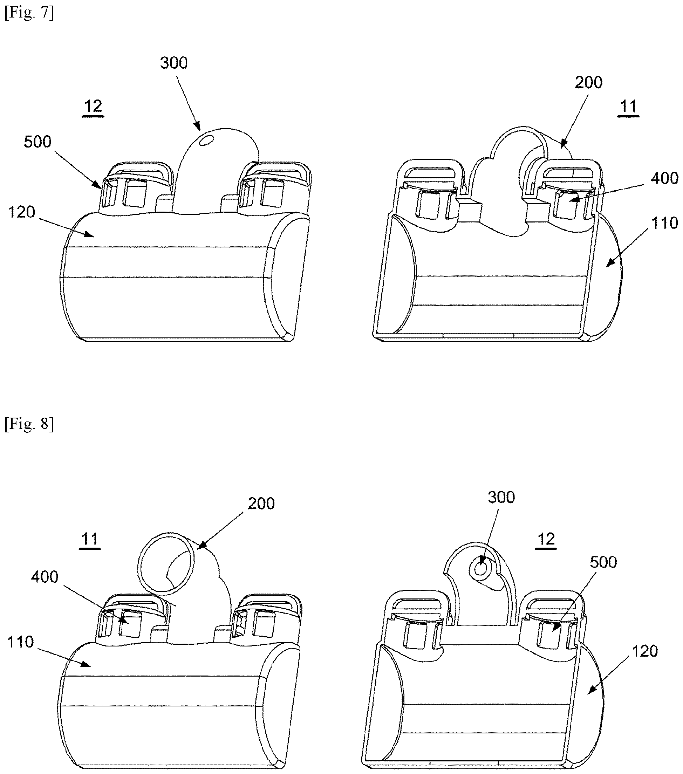

As illustrated in FIGS. 5 and 8, an insertion hole formed as an internal protruding type and coupled to the oxygen supply tube or suction tube may be formed to prevent sputum from being discharged to the outside of the oxygen supply and suction tube coupling part 300. The internal protruding type is not a type protruding to the outside and coupled to the oxygen supply tube or suction tube and is a type which is formed inside and to which the oxygen supply tube or suction tube is insertion-coupled. In addition, since the insertion hole is formed in the internal protruding type to have a guide tube shape, there are effects that the oxygen supply tube or suction tube formed of a substantially elastic material is prevented from bending or sagging toward the sputum accommodation part 100 and is smoothly guided toward the tracheostomy tube, that is, in the direction d1.

In addition, the present invention may further include an air entrance 500 including holes 510 and 520 open in the second direction d2 to smoothly supply air to the patient. In this case, the air entrance 500 may be formed with a grid structure and the like to prevent, except air, foreign substances including insects, such as mosquitoes and flies, and dust from being introduced into bronchi.

In addition, the air entrance 500 may not include the holes 510 and 520 open in the second direction d2 and may include one or more holes positioned at a level higher than the level of the maximum sputum accommodating position in the sputum accommodation part 100 to allow air to flow in or out therethrough. In addition, as described above, the sputum backflow prevention part 400 basically serves as an air entrance in addition to serving to prevent the backflow of sputum.

### (Method of manufacturing sputum container for tracheostomy patient).

FIGS. 5 and 6 are plan views of a first portion 11 and a second portion 12 for manufacturing the sputum container 10 for a tracheostomy patient according to the present invention, wherein FIG. 5 is a plan view as seen in the direction d1, and FIG. 6 is a plan view as seen in the direction d2, FIGS. 7 and 8 are perspective views of the first portion 11 and the second portion 12 for manufacturing the sputum container 10 for a tracheostomy patient according to the present invention, wherein FIG. 7 is a perspective view as seen in the direction d2, and FIG. 8 is a perspective view as seen in the direction d1.

As illustrated in FIGS. 5 to 8, the sputum container 10 for a tracheostomy patient may be formed by injection molding and coupling two portions which are the first portion 11 and the second portion 12, and for example, the first portion 11 and the second portion 12 may be ultrasonic welded to each other or mechanically coupled using protrusion and groove structures of the first portion 11 and the second portion 12.

In the case of the ultrasonic welding, a method of using a protrusion (protruding part) formed at any one or both sides of surfaces of the first portion 11 and the second portion 12 may be used so that the protrusion (protruding part) is melted and fused in a state in which the first portion 11 and the second portion 12 are in contact with each other.

In this case, for convenience in ultrasonic welding, the first portion and the second portion may be divided along a bent portion in the tracheostomy tube coupling part having a shape bent in the first direction, and a vertically divided portion may be formed in a region outside the bent portion so that it can be easy to perform ultrasonic welding. In addition, there are many ways to divide the first portion and the second portion.

### (Features of apparatus)

Hereinafter, features of the apparatus according to the present invention will be described.

### 1. Sputum reception.

Since an apparatus according to the present invention may be designed to be installed (coupled) to the tracheostomy tube to directly receive sputum which is discharged and expelled at a high speed or continuously flows through a tube inserted into the body regardless of the will of a patient due to characteristics of a tracheostomy patient, infection of patients and the harm delivered by sputum to medical workers (doctors, nurses, therapists, and caregivers) can be prevented. In addition, a current medical environment, in which there is a lot of sputum splattering in hospital rooms and treatment rooms, can be improved.

### 2. Breathable.

Since a tracheostomy patient directly breathes through a bronchus, breathing should not be disturbed while receiving the sputum. To this end, since the apparatus according to the present invention is developed to allow smooth oxygen supply while being used, the apparatus according to the present invention may receive the sputum and allow breathing at the same time. A hole, that is, the air entrance 500, is provided in the oxygen supply and suction tube coupling part which is connected to an external oxygen supply apparatus, and external air is normally supplied to the respiratory tract of the patient through the air entrance, the apparatus according to the present invention, and the tracheostomy tube. In addition, the holes 410 and 420 of the sputum backflow prevention part 400 also normally serve to allow air to flow in or out therethrough in addition to serving to prevent the backflow of the sputum. In addition, the apparatus according to the present invention is an apparatus designed in consideration of all situations in which the sputum strongly splatters or weakly flows.

### 3. No backflow of sputum

The apparatus according to the present invention is an apparatus developed with a unique design for preventing sputum from flowing back into a tube when an amount of the sputum contained in the apparatus according to the present invention is increased.

Since most patients using the present apparatus are lying down rather than standing, or lying rather than standing for most of the time, there is a risk that the sputum flows back into the tracheostomy tube (not shown) in the lying state.

The holes 410 and 420 of the sputum backflow prevention part 400 are provided to prevent this. Since the sputum is discharged first through a hole of the sputum backflow prevention part even in a situation in which the patient is lying down, or the sputum fills up, and the sputum flows back, breathing difficulties which may occur due to the backflow of the sputum can be prevented. That is, since the sputum is discharged to a sputum backflow prevention hole before the sputum flows back, fundamentally, the sputum does not flow in the tracheostomy tube.

In addition, the present invention may include a structure including a partition (partition wall) formed between an inner space connected to the tracheostomy tube and an inner space in which the sputum backflow prevention part hole is installed to prevent a situation in which the sputum flows into the tracheostomy tube when the sputum backflows. However, the inner partition wall may also not be included.

In this case, when it is assumed that there is a virtual plane between the inner space in which the hole of the sputum backflow prevention part is provided and the inner space connected to the tracheostomy tube, the partition wall may be formed on only 1/2 of the virtual plane, and the remaining 1/2 thereof may be open, and thus a situation in which air does not flow into the tracheostomy tube through the holes 510 and 520 of the air entrance 500 can be prevented.

In this case, when a mold is manufactured in consideration of injection molding of the apparatus according to the present invention, the partition wall may be formed at 1/2 (at a side at which the hole of the sputum backflow prevention part is provided) of a vertical cross-section of the apparatus according to the present invention, an open structure may be formed so that the partition wall is formed at the remaining 1/2 (at a side at which the hole of the air entrance) of the vertical cross-section to form an open structure, and the partition wall and the open structure may be coupled to each other to improve manufacturing convenience.

### 4. Structural points

A structure is provided with a design in which a portion receiving sputum and a portion through which oxygen flows are divided and allows breathing even in a situation the sputum is continuously received. The internal protruding type tube insertion hole is designed so that the oxygen supply tube is inserted into the oxygen supply and suction tube coupling part 300 of the apparatus according to the present invention in the case of an emergency in which saturation of percutaneous oxygen (SpO2) of a patient decreases. In the case of an external protruding type, since there is a risk that sputum is discharged therethrough, the internal protruding type is formed.

The present invention may also have a unique inner divider (partition wall or partition) structure which prevents a situation in which sputum flows into the airway and does not interfere with breathing even when sputum fills up. A safety hole of the sputum backflow prevention part for preventing the backflow of fully filled sputum has a design which allows the sputum to be discharged to the outside of the container before the sputum backflows.

The grid structure of the air entrance 500 through which oxygen flows therein is a structure capable of preventing, except oxygen, foreign substances (insects including mosquitoes and flies, dust, and the like) from being introduced into the bronchi.

### (Expected effects)

Hereinafter, expected effects of the apparatus according to the present invention will be described.

### 1. Infection management

Sputum can be prevented from being transferred to medical workers (doctors, nurses, therapists, and caregivers) when a patient discharges the sputum. Contaminants spreading in hospital rooms and other environments can be prevented. Grievances of the medical workers such as the doctors and the nurses are addressed, and a problem of secondary infection is also prevented. Foreign substances are prevented from being introduced from the outside. The above can be applied in terms of infection control when evaluating hospital accreditation.

### 2. Patient management

The number of suctions decreases. Sputum can be prevented from flowing on the skin to minimize skin troubles. Due to a warm humidification effect, the sputum is prevented from hardening, coughing is prevented, and breathing becomes easy. A hole of the oxygen supply and suction tube coupling part 300 which is connected to an oxygen line is formed therein. The partition (partition wall) may also be designed in a product for preventing backflow of the sputum.

### 3. Convenience

The present invention can replace a conventional technology or practice in which a disposal product including a paper cup or gauze is used. An ergonomic design is applied so that it is convenient to apply the present invention to a patient. It is easy to use when moving the patient. A plurality of holes are designed so that sputum can be discharged to the outside when the sputum is contained to an amount greater than or equal to a proper amount. This product is the world's first product which allows discharge of sputum and breathing at the same time.

As described above, although the present invention has been described with reference to specific embodiments and drawings, the present invention is not limited thereto.

### [Reference Numerals]

10: SPUTUM CONTAINER FOR A TRACHEOSTOMY PATIENT
100: SPUTUM ACCOMMODATION PART
200: TRACHEOSTOMY TUBE COUPLING PART
300: OXYGEN SUPPLY AND SUCTION TUBE COUPLING PART
400: SPUTUM BACKFLOW PREVENTION PART
410, 420: HOLE OF SPUTUM BACKFLOW PREVENTION PART
500: AIR ENTRANCE
510, 520: HOLE OF AIR ENTRANCE
600: COMMUNICATION PART

## Claims

1. A sputum container (10) for a tracheostomy patient, which is applied to a tracheostomy patient, the sputum container (10) comprising:
a sputum accommodation part (100) which accommodates sputum of a patient; and
a tracheostomy tube coupling part (200) which is provided at one upper side of the sputum accommodation part (100), is coupled to a tracheostomy tube which is a tracheostomy region of the patient and is disposed in a first direction (d1), receives the sputum of the patient, and delivers the sputum to the sputum accommodation part (100);
**characterized in that** the sputum container (10) further comprises
an oxygen supply and suction tube coupling part (300) which is provided at one side of the tracheostomy tube coupling part (200) in a second direction (d2), which is a direction opposite to the first direction (d1), and coupled to an oxygen supply tube or suction tube.

2. The sputum container (10) of claim 1, wherein the tracheostomy tube coupling part (200) is formed to extend upward from the sputum accommodation part (100) and has a shape bent in the first direction (d1).

3. The sputum container (10) of claim 1 or 2, further comprising a sputum backflow prevention part (400) which is formed to extend upward from the sputum accommodation part (100) and includes one or more holes (410, 420) which are open in the first direction (d1) to prevent the sputum contained in the sputum accommodation part (100) from flowing backward and allow air to flow into or out of the sputum container (10).

4. The sputum container (10) of claim 3, wherein the sputum backflow prevention part (400) is provided as one or more sputum backflow prevention parts (400) formed at each side of the tracheostomy tube coupling part (200) on the sputum accommodation part (100).

5. The sputum container (10) of claim 1, wherein:
the tracheostomy tube coupling part (200) is formed on an extension part formed to extend upward from the sputum accommodation part (100) in the first direction (d1);
the oxygen supply and suction tube coupling part (300) is formed on the extension part formed to extend upward from the sputum accommodation part (100) in the second direction (d2); and
the tracheostomy tube coupling part (200) and the oxygen supply and suction tube coupling part (300) are integrally formed.

6. The sputum container (10) of claim 5, wherein an insertion hole which is formed as an internal protruding type and into which an oxygen supply tube or suction tube is inserted is formed to prevent the sputum from being discharged to outside of the oxygen supply and suction tube coupling part (300).

7. The sputum container (10) of any one of claims 1 to 6, further comprising an air entrance (500) including a hole (510, 520) which opens in the second direction (d2) to smoothly supply air to the patient.

8. The sputum container (10) of claim 7, wherein the air entrance (500) is formed as a grid structure to prevent, except air, foreign substances including insects, such as flies and mosquitos, and dust from being introduced into bronchi.

9. The sputum container (10) of any one of claims 1 to 8, wherein the sputum accommodation part (100) has a cylindrical shape of which a cross-section has a circular shape.

10. The sputum container (10) of claim 3 or 4, comprising a communication part (600) which is disposed between the sputum backflow prevention part (400) and the tracheostomy tube coupling part (200) and has a structure through which at least parts of the sputum backflow prevention part (400) and the tracheostomy tube coupling part (200), which are positioned at levels higher than a level of a maximum sputum accommodating position in the sputum accommodation part (100), communicate with each other so that the air flowing into through the sputum backflow prevention part (400) flows into an airway of the patient through the tracheostomy tube coupling part (200) even when the sputum is maximally contained in the sputum accommodation part (100).

11. The sputum container (10) of claim 10, wherein the maximum sputum accommodating position is a position of a hole having a lowest height from the sputum accommodation part (100) among the holes which are open in the sputum accommodation part (100) in the first direction (d1).

## Patentansprüche

1. Ein Sputumbehälter (10) für einen Tracheotomiepatienten, der bei einem Tracheotomiepatienten angewendet wird, wobei der Sputumbehälter (10) umfasst:
einen Sputumaufnahmeteil (100), der das Sputum eines Patienten aufnimmt; und
einen Tracheotomie-Tubus-Kupplungsteil (200), der an einer oberen Seite des Sputumaufnahmeteils (100) vorgesehen ist, mit einem Tracheotomie-Tubus gekoppelt ist, der den Tracheotomie-Bereich des Patienten darstellt, in einer ersten Richtung (d1) angeordnet ist, das Sputum des Patienten empfängt und es zum Sputumaufnahmeteil (100) weiterleitet;
**dadurch gekennzeichnet, dass** der Sputumbehälter (10) ferner umfasst:
einen Sauerstoff- und Absaugrohr-Kupplungsteil (300), der an einer Seite des Tracheotomie-Tubus-Kupplungsteils (200) in einer zweiten Richtung (d2), die der ersten Richtung (d1) entgegengesetzt ist, vorgesehen ist und mit einem Sauerstoffzuführungs- oder Absaugrohr gekoppelt ist.

2. Der Sputumbehälter (10) nach Anspruch 1, wobei der Tracheotomie-Tubus-Kupplungsteil (200) so ausgebildet ist, dass er sich von dem Sputumaufnahmeteil (100) nach oben erstreckt und eine in die erste Richtung (d1) gebogene Form aufweist.

3. Der Sputumbehälter (10) nach Anspruch 1 oder 2, weiter umfassend einen Rückflussverhinderungsabschnitt (400), der sich vom Sputumaufnahmeteil (100) nach oben erstreckt und ein oder mehrere Löcher (410, 420) enthält, die in der ersten Richtung (d1) geöffnet sind, um das im Sputumaufnahmeteil (100) enthaltene Sputum am Rückfluss zu hindern und Luft in oder aus dem Sputumbehälter (10) strömen zu lassen.

4. Der Sputumbehälter (10) nach Anspruch 3, wobei der Rückflussverhinderungsabschnitt (400) als ein oder mehrere Rückflussverhinderungsabschnitte (400) vorgesehen ist, die an jeder Seite des Tracheotomie-Tubus-Kupplungsteils (200) auf dem Sputumaufnahmeteil (100) ausgebildet sind.

5. Der Sputumbehälter (10) nach Anspruch 1, wobei:
der Tracheotomie-Tubus-Kupplungsteil (200) auf einem Erweiterungsteil ausgebildet ist, der sich vom Sputumaufnahmeteil (100) in der ersten Richtung (d1) nach oben erstreckt;
der Sauerstoff- und Absaugrohr-Kupplungsteil (300) auf dem Erweiterungsteil ausgebildet ist, der sich vom Sputumaufnahmeteil (100) in der zweiten Richtung (d2) nach oben erstreckt; und
der Tracheotomie-Tubus-Kupplungsteil (200) und der Sauerstoff- und Absaugrohr-Kupplungsteil (300) integral ausgebildet sind.

6. Der Sputumbehälter (10) nach Anspruch 5, wobei eine Einführungsöffnung, die als interne Vorsprungsstruktur ausgebildet ist und in die ein Sauerstoffzufuhr- oder Absaugrohr eingeführt wird, ausgebildet ist, um zu verhindern, dass das Sputum aus dem Sauerstoff- und Absaugrohr-Kupplungsteil (300) nach außen austritt.

7. Der Sputumbehälter (10) nach einem der Ansprüche 1 bis 6, weiter umfassend einen Lufteinlass (500), der ein Loch (510, 520) umfasst, das in der zweiten Richtung (d2) geöffnet ist, um Luft reibungslos zum Patienten zuzuführen.

8. Der Sputumbehälter (10) nach Anspruch 7, wobei der Lufteinlass (500) als Gitterstruktur ausgebildet ist, um das Eindringen von Fremdkörpern, einschließlich Insekten wie Fliegen und Mücken, sowie Staub in die Bronchien zu verhindern, mit Ausnahme von Luft.

9. Der Sputumbehälter (10) nach einem der Ansprüche 1 bis 8, wobei der Sputumaufnahmeteil (100) eine zylindrische Form mit einem kreisförmigen Querschnitt aufweist.

10. Der Sputumbehälter (10) nach Anspruch 3 oder 4, umfassend einen Kommunikationsabschnitt (600), der zwischen dem Rückflussverhinderungsabschnitt (400) und dem Tracheotomie-Tubus-Kupplungsteil (200) angeordnet ist und eine Struktur aufweist, durch die mindestens Teile des Rückflussverhinderungsabschnitts (400) und des Tracheotomie-Tubus-Kupplungsteils (200), die auf höheren Ebenen als die maximale Sputum-Aufnahmeposition im Sputumaufnahmeteil (100) positioniert sind, miteinander kommunizieren, sodass die durch den Rückflussverhinderungsabschnitt (400) einströmende Luft durch den Tracheotomie-Tubus-Kupplungsteil (200) in die Atemwege des Patienten gelangt, selbst wenn das Sputum maximal im Sputumaufnahmeteil (100) enthalten ist.

11. Der Sputumbehälter (10) nach Anspruch 10, wobei die maximale Sputum-Aufnahmeposition die Position eines Lochs ist, das die niedrigste Höhe vom Sputumaufnahmeteil (100) aufweist, unter den Löchern, die in der ersten Richtung (d1) im Sputumaufnahmeteil (100) geöffnet sind.

## Revendications

1. Un récipient à expectorations (10) pour un patient trachéotomisé, qui est appliqué à un patient trachéotomisé, le récipient à expectorations (10) comprenant :
une partie d'accueil des expectorations (100) qui accueille les expectorations d'un patient ; et
une partie de raccordement au tube de trachéotomie (200) qui est située sur un côté supérieur de la partie d'accueil des expectorations (100), est raccordée à un tube de trachéotomie dans la région de la trachéotomie du patient et est disposée dans une première direction (d1), reçoit les expectorations du patient et les transfère à la partie d'accueil des expectorations (100) ;
**caractérisé en ce que** le récipient à expectorations (10) comprend en outre :
une partie de raccordement à un tube d'oxygène et d'aspiration (300) située sur un côté de la partie de raccordement au tube de trachéotomie (200) dans une seconde direction (d2), qui est opposée à la première direction (d1), et raccordée à un tube d'oxygène ou à un tube d'aspiration.

2. Le récipient à expectorations (10) selon la revendication 1, dans lequel la partie de raccordement au tube de trachéotomie (200) est formée pour s'étendre vers le haut depuis la partie d'accueil des expectorations (100) et présente une forme incurvée dans la première direction (d1).

3. Le récipient à expectorations (10) selon la revendication 1 ou 2, comprenant en outre une partie anti-reflux d'expectorations (400) formée pour s'étendre vers le haut depuis la partie d'accueil des expectorations (100) et comprenant un ou plusieurs trous (410, 420) ouverts dans la première direction (d1) pour empêcher le reflux des expectorations contenues dans la partie d'accueil des expectorations (100) et permettre à l'air de circuler dans ou hors du récipient à expectorations (10).

4. Le récipient à expectorations (10) selon la revendication 3, dans lequel la partie anti-reflux d'expectorations (400) est prévue comme une ou plusieurs parties anti-reflux d'expectorations (400) formées de chaque côté de la partie de raccordement au tube de trachéotomie (200) sur la partie d'accueil des expectorations (100).

5. Le récipient à expectorations (10) selon la revendication 1, dans lequel :
la partie de raccordement au tube de trachéotomie (200) est formée sur une partie d'extension s'étendant vers le haut depuis la partie d'accueil des expectorations (100) dans la première direction (d1) ;
la partie de raccordement à un tube d'oxygène et d'aspiration (300) est formée sur la partie d'extension s'étendant vers le haut depuis la partie d'accueil des expectorations (100) dans la seconde direction (d2) ; et
la partie de raccordement au tube de trachéotomie (200) et la partie de raccordement à un tube d'oxygène et d'aspiration (300) sont formées de manière intégrée.

6. Le récipient à expectorations (10) selon la revendication 5, dans lequel un trou d'insertion, formé comme un type interne saillant et dans lequel un tube d'oxygène ou un tube d'aspiration est inséré, est formé pour empêcher l'expulsion des expectorations à l'extérieur de la partie de raccordement à un tube d'oxygène et d'aspiration (300).

7. Le récipient à expectorations (10) selon l'une des revendications 1 à 6, comprenant en outre une entrée d'air (500) comprenant un trou (510, 520) ouvert dans la seconde direction (d2) pour fournir de l'air de manière fluide au patient.

8. Le récipient à expectorations (10) selon la revendication 7, dans lequel l'entrée d'air (500) est formée comme une structure en grille pour empêcher, à l'exception de l'air, l'introduction de substances étrangères, y compris des insectes tels que les mouches et les moustiques, et de la poussière dans les bronches.

9. Le récipient à expectorations (10) selon l'une des revendications 1 à 8, dans lequel la partie d'accueil des expectorations (100) a une forme cylindrique dont une section transversale est de forme circulaire.

10. Le récipient à expectorations (10) selon la revendication 3 ou 4, comprenant une partie de communication (600) qui est disposée entre la partie anti-reflux d'expectorations (400) et la partie de raccordement au tube de trachéotomie (200) et a une structure par laquelle au moins une partie de la partie anti-reflux d'expectorations (400) et de la partie de raccordement au tube de trachéotomie (200), positionnées à des niveaux plus élevés que le niveau d'une position maximale d'accueil des expectorations dans la partie d'accueil des expectorations (100), communiquent entre elles de manière à ce que l'air entrant par la partie anti-reflux d'expectorations (400) pénètre dans une voie respiratoire du patient par la partie de raccordement au tube de trachéotomie (200), même lorsque les expectorations sont contenues au maximum dans la partie d'accueil des expectorations (100).

11. Le récipient à expectorations (10) selon la revendication 10, dans lequel la position maximale d'accueil des expectorations est une position d'un trou ayant la hauteur la plus basse depuis la partie d'accueil des expectorations (100) parmi les trous ouverts dans la partie d'accueil des expectorations (100) dans la première direction (d1).
